(19) Europäisches Patentamt

European Patent Office

Office européen des brevets

(11) Publication number: **0 297 379 B1**

# EUROPEAN PATENT SPECIFICATION

(45) Date of publication of patent specification: **19.08.92**

(51) Int. Cl.⁵: **C12Q 1/68**

(21) Application number: **88109769.5**

(22) Date of filing: **20.06.88**

(54) **Method for amplifying genes.**

(30) Priority: **30.06.87 US 68671**

(43) Date of publication of application:
**04.01.89 Bulletin 89/01**

(45) Publication of the grant of the patent:
**19.08.92 Bulletin 92/34**

(84) Designated Contracting States:
**DE FR GB**

(56) References cited:
**EP-A- 0 184 056**
**EP-A- 0 192 168**
**EP-A- 0 200 362**

(73) Proprietor: **MILES INC.**
**One Mellon Center 500 Grant Str.**
**Pittsburgh, PA 15219-2502(US)**

(72) Inventor: **Dattagupta, Nanibhushan**
**470 Prospect Street**
**New Haven, CT 06511(US)**

(74) Representative: **Dänner, Klaus, Dr. et al**
**c/o Bayer AG Konzernverwaltung RP Patente**
**Konzern**
**W-5090 Leverkusen 1 Bayerwerk(DE)**

EP 0 297 379 B1

**Description**

BACKGROUND OF THE INVENTION

Field of the Invention

The present invention relates to the amplification of a gene for the detection of specific sequences. More particularly, the present invention concerns a method of detecting a specific nucleic acid sequence using immobilized oligonucleotides.

Background Information

Large scale production of a nucleic acid sequence is usually done by cloning the particular sequence in a specific vector. The sequence to be cloned is isolated, identified, coupled covalently to a single or double-stranded vector and then cloned. The vectors with the extra DNA are separated from the host cell and, depending on the requirements, the cloned piece of DNA has to be restricted and separated from the rest of the DNA. If one requires single-stranded DNA, either it is cloned in a single-stranded vector or strand separation is necessary. All these techniques involve skilled manipulation of biochemical and biological systems.

Analytical Biochemistry, 140, 95-103, (1984) describes a method of producing DNA hybridization probes by non-specifically immobilizing a single strand DNA template to cellulose. Although the method is useful, the length distribution of the newly synthesized product DNA is not as uniform as might be desired. It now appears this may be due to multiple attachments of the template DNA to the cellulose.

A homogenous system involving two probes has been described in EP-A-192 168. This method uses two non-overlapping probes, one of which is labeled for detection and the other for the separation of the hybrid. The assay takes place in a homogeneous solution and the hybrid is subsequently separated by an immobilization reaction with a solid support and a separation probe.

Amplification of purified polynucleotide sequences by using an immobilized polynucleotide and oligonucleotide as a primer has been described in European Patent Application 184,056 and in Ashley et al, Anal. Biochem., supra. In EP 184,056, an end coupling and in Ashley et al nonspecific random coupling reactions were used for the immobilization of the polynucleotides.

Amplification in solution after amplification analysis requires immobilization or other methods, e.g., gel electrophoresis with labeled materials.

Saiki et al.(Science,230,1350, (1985) and EP-A2-200 362) described a method of amplification of a beta-globin nucleic acid sequence in a human genomic sample for the detection of point mutation by hybridization with an oligonucleotide This product sequence is formed in solution. For hybridization, the DNA has to be either immobilized after amplification or a restriction digestion and separation must be carried out for analysis of the sequence.

SUMMARY OF THE INVENTION

The present invention relates to the amplification of a gene for the detection of specific sequences. The amplified sequences are produced by using immobilized primer sequences so that the product is formed in an immobilized state. The sequences which serve as primers may carry labels or may be labeled in the extending sequence.

More particularly, the present invention concerns a method for amplifying a specific target DNA sequence in a sample wherein

(a) the sample is contacted under hybridization conditions with two primer nucleic acids, each being complementary to a strand of the DNA sequence to be amplified,

(b) the resultant product from step (a) is contacted with an extender enzyme and at least one nucleic acid residue under conditions to elongate the resultant hybridized primer nucleic acids,

(c) the product of (b) is denatured, and

(d) a cycle of steps (a), (b) and (c) is repeated at least once with the product of step (c) of the previous cycle being used in place of the sample, characterised in that the primer nucleic acid are in an immobilised form.

The present invention further concerns a kit for amplifying a specific target DNA sequences, the kit comprising one or more containers containing (a) immobilized primer nucleic acids, (b) an extender enzyme and (c) at least one nucleic acid residue.

The kit can be brought into a form comprising one or more containers containing (a) immobilized nucleic acids, (b) an extender enzyme and (c) at least one nucleic acid residue and (d) a labeled hybridizable oligonucleotide specific for the detection of the amplified sequence.

The present invention is surprising and unexpected in that an immobilized oligonucleotide can be used for amplification of a specific sequence in a crude mixture containing many other sequences. Moreover, the desired analyte produced at the end of the process is in immobilized form and ready for heterogeneous phase assays.

The present invention produces immobilized amplified sequences.

The present invention can be processed as an automated system.

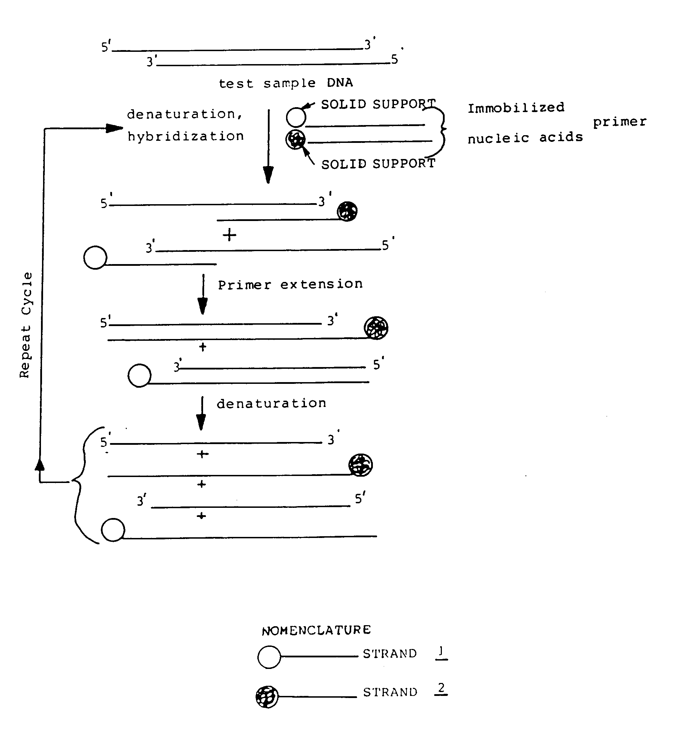

## BRIEF DESCRIPTION OF THE DRAWING

The figure is a schematic diagram showing the steps to carry out an amplification according to the present invention.

## DETAILED DESCRIPTION OF THE INVENTION

The present invention concerns amplification of a test DNA sequence for an assay by contacting one nucleic acid attached to a solid support complementary to one strand and another nucleic acid attached to a solid support complementary to another strand of the test sample nucleic acid under hybridization conditions, extending the nucleic acid with an enzyme such that sequences complementary to the test sample are produced, denaturing the product and reannealing to produce a more extendable structure and repeating the process to produce a solid support immobilized test sequence in an amount greater than the starting concentration. The product is then assayed, for example, as follows:

(1) by hybridization with a specific probe;

(2) if during an extension reaction a labeled nucleic acid residue is incorporated, the extent of incorporation of such a residue determines the presence of the specific sequence; or

(3) a post extension agglutination reaction.

As described above, Saiki et al described a method of amplification of a test sample. Saiki et al used two different oligonucleotides as primers. The test sample nucleic acid served as a template for the primer extension reaction. The oligonucleotides were complementary to the ends of the two strands of a double stranded sample DNA. In Saiki et al, one oligonucleotide is specific for one strand and the other oligonucleotide is specific for the complementary strand. Both oligonucleotides flank the region of mutation sequence to be analyzed. When the oligonucleotides are mixed with the test sample nucleic acids under annealing conditions, the oligonucleotides will hybridize in a fashion such that the 3' hydroxyl of each oligonucleotide will be available for extension. After the oligonucleotide is extended with template dependent primer extension enzyme, e.g., DNA, polymerase, Reverse transcriptase etc., the processes of oligonucleotide hybridization and extension are repeated. The disadvantage of such an amplification is that the final product analysis requires difficult sample handling steps, e.g., immobilizing.

The present invention uses immobilized nucleic acid primers. The final amplified products of the present invention are already immobilized.

In the figure, the test sample nucleic acid, which serves as a template for the initial cycle of amplification, is contacted with immobilized primer nucleic acids under conditions of hybridization. One primer sequence is specific for one strand and the other is specific for the complementary strand. The primers are not complementary to each other. After the primer template hybrids are formed they are extended by an enzyme. The amplified sequence is then recycled for further amplification.

The primer nucleic acids are any hybridizable DNA, RNA or oligonucleotides.

In a preferred embodiment, the primer nucleic acids are oligonucleotides and they are complementary to the 3' end sequences of the sample DNA. The nucleic acid primers can be of any length between 3 bases and 10 kilobases and greater, preferably between 5 and 100 bases. Any complementary (to the sample) nucleic acids with 3' - hydroxy can serve as primer for template mediated extension reaction.

The objective of using two printers is to amplify both strands. The primers do not have to be complementary (in their entirety), to the sample sequence. They should be complementary enough to initiate sequence specific, template mediated extension reaction. In order to maintain the fidelity of the template mediated primer extension reaction the initiation should occur from a perfectly matched base paired region. The minimum sequence may include 3 base pairs. Usually a 5 to 7 base pair double stranded region is strong enough to undergo primer intension reaction at 25°C and below. If it is less than 5 bp, a low temperature extension reaction has been carried out.

The present invention is an improvement over the amplification methods described in the prior art. The present invention describes a method of amplification of test sample nucleic acids where the amplified sequence is produced in an immobilized form. The major advantage associated with such process is that the product is formed in an immobilized state. This makes the separation from the unamplified sequence (sometimes $10^3$-$10^6$ times the test sequence) easier. Since it is in an immobilized form the analysis is easier.

Immobilization of a nucleic acid, especially an oligonucleotide is described in Affinity Chromatography, Herbert Schott, Marcel Dekker, Inc., pages 15 to 21. Most of the immobilization reactions described therein can be carried out via spacer or linker residues. For example, instead of using "SEPHADEX$^{(R)}$" or cellulose particles directly, it is possible to use polyamine or protein linked "SEPHADEX$^{(R)}$" or cellulose and use the protein or polyamine residues as the coupling sites for an oligonucleotide.

For example, cyanogen bromide activated "SEPHADEX$^{(R)}$" can first be reacted with a large excess of a polyamine, e.g., spermine, and then coupled to an oligonucleotide by an oxidative reaction. As has been described in EP 164,586, aminolabeled oligonucleotides can be prepared with variable linker lengths. Other methods for preparing aminolabeled oligonucleotides have also been described in the art.

As an immobilization matrix (solid support) for the present invention reactive or activated cellulose, "SEPHADEX®", "SEPHAROSE®" "SEPHACRYL®", polystyrene latex, polyacrylates, polyvinylalcohols, or other synthetic or naturally occurring matrices which can be activation to undergo chemical reaction.

The substrate for the extension reaction is one or more nucleic acid residues, e.g., nucleoside triphosphate (NTP).

Since the addition of nucleoside residues to the primer or extension reaction necessary for the fidelity of the sequence should be specific, enzymes such as DNA polymerase, a Klenow fragment of polymerase or reverse transcriptase, are preferable. These enzymes catalyze a specific primer extension reaction. It is surprising that such enzymes can act on oligonucleotide primers when they are immobilized.

The present invention produces immobilized sequences for analysis. The presence of such a sequence can be detected by using labeled nucleotide residues as substrates for extension. As, for example, if a $^{32}$P-labeled or fluorescein labeled nucleoside triphosphate is used as a substrate for extension with DNA polymerase the extended product will be labeled. By detecting such labels (radioactive for $^{32}$P and fluorescence for fluorescein) it is possible to ascertain the presence of the test sequence nucleic acid.

The amplified signal can be detected by hybridization with a specific probe.

The immobilized oligonucleotide probes will comprise at least one single stranded base sequence complementary to the 3' end or the 5' end of the sequence to be amplified. The probe should not be complementary to the entire sequence before the enzymatic extension reaction is conducted. The homology of the ends of the probe can be as short as three nucleotides and can be as long as 200 nucleotides, however, between 10 and 30 nucleotide residues is preferred.

The oligonucleotides can be immobilized by virtue of the oligonucleotides being linked at one end thereof to a solid substrate and wherein the elongation occurs in a direction opposite to the solid substrate.

The sample can comprise double stranded DNA and each oligonucleotide can be complementary to a strand of the double stranded DNA.

Detection of the presence of the amplified sequence can be conducted by any of the following:

(1) labeling the nucleic acid residue, for example, with a label, such as, for example, a radioactive moiety, a dyestuff, a fluorescent moiety or an enzyme, and then conducting an analysis to determine the presence of such label;

(2) subjecting the product of step (d) of the aforementioned method for detecting specific target nucleic acid sequences to hybridization conditions with a labeled probe (such label can be as described hereinabove) hybridizable with the sequence being tested for and determining if in fact hybridization has occurred;

(3) determination of a change or difference in a physical property, e.g., to detect a difference in molecular weight or density by conducting electrophoresis, centrifugation, gel permeation chromatography or using microscopy (visual determination to detect a difference in size).

The invention will now be described with reference to the following non-limiting examples.

Example 1:

The sequence of the human beta-globin gene is used as a model system for this invention. Any known sequence can be amplified by proper selection of the immobilized primers.

A sequence WP or shorter

WP    5' $\underline{AC}$ $\underline{AC}$ $\underline{AA}$ $\underline{CT}$ G $\underline{TGT}$ $\underline{TC}$ $\underline{A}$ $\underline{C}$ $\underline{T}$ $\underline{A}$ $\underline{GC}$ 3'

and a sequence CP or shorter

CP    3' $\underline{CC}$ $\underline{AC}$ $\underline{TT}$ $\underline{GC}$ $\underline{AC}$ $\underline{CT}$ $\underline{AC}$ $\underline{TT}$ $\underline{CA}$ $\underline{AC}$ 5'

are synthesized by a known oligonucleotide synthesis method. The oligonucleotides are then phosphorylated at the 5' end by an enzymatic method as described in Molecular Cloning, Maniatis et al, Cold Spring Harbor Laboratory, 125, (1982).

These oligonucleotides and their relationship to the target globin sequences have been published by Saiki et al, Science, 230, 1350, (1985). The phosphorylated oligonucleotides are then immobilized to beads according to methods described in Affinity Chromatography, H. Schott, Marcel Dekker publisher, 15, (1984).

The beads approximately 200 $\mu$l in aqueous suspension containing immobilized WP and CP are added to a solution (1 ml) containing 10 mM tris pH 7.5, 50 mM NaCl, 10 mM $MgCl_2$, 1.5 mM deoxynucleoside triphosphates (all four). They are heated to 37°C for 10 minutes and 20 units of Klenow fragment of E. coli DNA polymerase (PL-Biochemical) is added. The reaction is allowed to proceed for 5 minutes. Then the solution is centrifuged in a microfuge and using one of the deoxynucleoside triphosphate as the radioactive substrate the background reaction is estimated by counting the radioactivity of the beads in a scintilation counter.

The reaction is repeated after adding (0.1 $\mu$g) 10 $\mu$l aqueous heat denatured (100°C) ice cooled sample DNA (human). The cycle is repeated by heating the whole mixture in a boiling water bath and rapidly cooling to 37°C. For every cycle an aliquot of fresh enzyme (2 units) is added.

The final product is collected as immobilized amplified sequence by centrifugation in a microfuge. The immobilized product nucleic acid is then assayed in three different ways, namely, as follows:.

(1) hybridization with a labeled 19 mer;
(2) digestion with two enzymes (Science, 230, 1350 (1985));
(3) direct detection with or without any restriction digestion.

If one of the deoxy NTP's is labeled, e.g., with $^{32}$P direct counting of radioactivity before and after a restriction digestion will give the information about the sequence.

Example 2: Hybridization With a Labeled 19 mer Hybridizable With Labeled Oligonucleotide Probes

The amplified product immobilized onto beads is denatured by heating on a boiling water bath and then chilled in ice. As is described below, the beads are then hybridized with $^{32}$P-labeled oligonucleotides.

For molecular hybridization, the following mixture is prepared:

450 $\mu$l 20X NET buffer
750 $\mu$l 20% dextran sulfate
150 $\mu$l deionized water
75 $\mu$l 100X Denhardt's solution
75 $\mu$l 10% NP-40 detergent (Sigma)

To this mixture is added a one tenth portion of the oligonucleotide probe prepared as described in N. Dattagupta, D. Rabin, G. Michaud and P. M. M. Rae, "A Simple Method for Generation of High Specific Activity Oligonucleotide Probes", BioTechniques, Vol. 5, No. 1, 38-43, (1987). The final 19-mer probe concentration is approximately 1 nanomolar. It has been found that addition of more probe than this results in an increased background. To prepare a labeled probe for a single or a few reactions, the labeling protocol detailed above can be scaled down, using proportionally less primer, template, dGTP, and dATP. A 0.1X reaction can be done in 10 $\mu$l, a 0.2X reaction can be done in 20 $\mu$l, etc. When volume permits, the [alpha$^{32}$P]-alpha-ATP need not be evaporated to dryness.

Beads containing amplified approximately 0.5 ml of the sequence are placed in plastic bags (e.g., "SEAL-A-MEAL$^{(R)}$", "SEAL AND SAVE$^{(R)}$", etc) with one end left open. The radioactive hybridization mix is added with a Pasteur pipet or a syringe with a 21 gauge needle and the remainder end of the bag is sealed. Hybridization is conducted for one hour at 50°C.

After hybridization, the beads are separated by centrifugation in test tubes and washed with 6X SSC for 15 minutes with gentle shaking.

For stringency washing, the beads are transferred to a prewarmed 1.5 ml culture tube containing 1.4 ml 6X SSC. The tubes are replaced in a 57°C circulating water bath for 10 minutes. The liquid is centrifuged quickly, another 1.5 ml prewarmed 6X SSC is added, the tubes are then replaced in the water bath for another 10 minutes, the liquid is centrifuged again and the supernatant liquid is then discarded.

The radioactivity in the tube is counted in a scintillation counter. The radioactivity associated with the beads is the measure of hybridization and hence the indication of the presence of test gene in the original sample.

Example 3: Digestion With Two Enzymes

As has been described by Saiki et al, Science, 230, 1350 (1985), the amplified product can be assayed by hybridization and then restriction digestion and separation by gel electrophoresis or thin layer chromatography.

Example 4: Direct Detection

The amplified product can be directly assayed by using a label carrying nucleoside triphosphate as the substrate for the amplification/extension reaction. As for example if a $^{32}$P-labeled or a fluorescein labeled or a biotin labeled nucleoside triphosphate is used as the substrate, the incorporation of the labels into the extended nucleic acids will be the direct indication of the specific processes and hence the presence of a specific test sequence. After the amplification, the amplified nucleic acids will be in beads and the presence of any label on the beads will indicate the presence of the test sequence. The assays for fluorescein, biotin or $^{32}$P are well known in the art. Other common labels can also be used for this purpose.

The difference in the physical properties of the beads can also be used for detection. Electrophoretic mobility, density in a centrifugation field and chromatographic properties can also be used for detection purposes.

**Claims**

1. A method for amplifying a specific target DNA sequence in a sample wherein (a) the sample is contacted under hybridization conditions with two primer nucleic acids, each being complementary to a strand of the DNA sequence to be amplified, (b) the resultant product from step (a) is contacted with an extender enzyme and at least one nucleic acid residue under conditions to elongate the resultant hybridized primer nucleic acids, (c) the product of step (b) is denatured, and (d) a cycle of steps (a), (b), and (c) is repeated at least once with the product of step (c) of the previous cycle being used in place of the sample,
    characterized in that the primer nucleic acids are in an immobilized form.

2. A method according to claim 1 wherein the primer nucleic acids are oligonucleotides.

3. A method according to claim 1 or 2 wherein the primer nucleic acids are immobilized by being linked to a solid substrate and wherein elongation occurs in a direction opposite to said substrate.

4. A kit for performing the method of claim 1 comprising one or more containers containing (a) said primer nucleic acids, (b) said extender enzyme, and (c) at least one nucleic acid residue,
    characterized in that the primer nucleic acids are in an immobilized form.

5. A kit according to claim 4 wherein the primer nucleic acids are oligonucleotides.

6. A kit according to claim 4 or 5 wherein the primer nucleic acids are immobilized by being linked to a solid substrate.

**Patentansprüche**

1. Verfahren zur Amplifikation einer spezifischen Ziel DNA Sequenz in einer Probe, worin
    (a) die Probe unter Hybridisierungsbedingungen mit zwei Primernukleinsäuren in Kontakt gebracht wird, wobei jede zu einem Strang der zu amplifizierenden DNA Sequenz komplementär ist,
(b) das erhaltene Produkt aus Schritt (a) mit einem Enzym zur Verlängerung und mindestens einem Nukleinsäurerest unter für die Elongation der erhaltenen hybridisierten Primernukleinsäure geeigneten Bedingungen in Kontakt gebracht wird,
(c) das Produkt aus Schritt (b) denaturiert wird und
(d) ein Zyklus aus den Schritten (a), (b) und (c) mindestens einmal mit dem Produkt aus Schritt (c) des vorhergehenden Zyklus anstelle der Probe wiederholt wird,
dadurch gekennzeichnet, daß die Primernukleinsäuren in immobilisierter Form vorliegen.

2. Verfahren nach Anspruch 1, worin die Primernukleinsäuren Oligonukleotide sind.

3. Verfahren nach Anspruch 1 oder 2, worin die Primernukleinsäuren durch Bindung an ein festes Substrat immobilisiert sind, und worin die Elongation in entgegengesetzter Richtung zum Substrat abläuft.

4. Kit zum Durchführen des Verfahrens nach Anspruch 1, enthaltend einen oder mehrere Behälter mit
(a) den Primernukleinsäuren,
(b) dem Enzym zur Verlängerung und
(c) mindestens einem Nukleinsäurerest,
dadurch gekennzeichnet, daß die Primernukleinsäuren in immobilisierter Form vorliegen.

5. Kit nach Anspruch 4, worin die Primernukleinsäuren Oligonukleotide sind.

6. Kit nach Anspruch 4 oder 5, worin die Primernukleinsäuren durch Bindung an ein festes Substrat immobilisiert sind.

**Revendications**

1. Procédé pour amplifier dans un échantillon une séquence spécifique d'ADN servant de cible, dans lequel (a) l'échantillon est mis en contact dans des conditions d'hybridation avec deux acides nucléiques servant d'amorces, chacun étant complémentaire d'un' brin de la séquence d'ADN à amplifier, (b) le produit résultant de l'étape (a) est mis en contact avec un enzyme servant d'agent d'extension et au moins un résidu d'acide nucléique dans des conditions permettant l'allongement des acides nucléiques d'amorces hybridés résultants, (c) le produit de l'étape (b) est dénaturé, et (d) un cycle des étapes (a), (b) et (c) est répété au moins une fois, le produit de l'étape (c) du cycle précédent étant utilisé à la place de l'échantillon,
caractérisé en ce que les acides nucléiques servant d'amorces sont présents sous une forme immobilisée.

2. Procédé suivant la revendication 1, dans lequel les acides nucléiques servant d'amorces sont des oligonucléotides.

3. Procédé suivant la revendication 1 ou 2, dans lequel les acides nucléiques servant d'amorces sont immobilisés par liaison à un substrat solide et un allongement se produit dans le sens opposé à celui dudit substrat.

4. Kit pour la mise en oeuvre du procédé suivant la revendication 1, comprenant un ou plusieurs récipients contenant (a) les acides nucléiques servant d'amorces, (b) l'enzyme servant d'agent d'extension, et (c) au moins un résidu d'acide nucléique,
caractérisé en ce que les acides nucléiques servant d'amorces sont présents sous une forme immobilisée.

5. Kit suivant la revendication 4, dans lequel les acides nucléiques servant d'amorces sont des oligonucléotides.

6. Kit suivant la revendication 4 ou 5, dans lequel les acides nucléiques servant d'amorces sont immobilisés par liaison à un substrat solide.